# EUROPEAN PATENT APPLICATION

(11) **EP 2 604 995 A1**
(43) Date of publication of application: **19.06.2013**
(21) Application number: 11193929.4
(22) Date of filing: 16.12.2011
(51) Int. Cl.: G01N 1/22, G01N 33/15

(54) **Content extractor for metered dose inhalers**

(71) Applicant: Deva Holding Anonim Sirketi, 34303 Istanbul (TR)
(72) Inventor: Haas, Philipp Daniel, 34303 ISTANBUL (TR); Koc, Fikret, 34303 ISTANBUL (TR); Sunel, Fatih, 34303 ISTANBUL (TR); Sivasligil, Ramazan, 34303 ISTANBUL (TR)

(57) **Abstract**

This invention relates to an aerosol can sample content extractor apparatus for determining of drug content (assay) and water content or moisture content of aerosol formulation from pressurized metered dose inhalers (MDI). The device allows the accurate determination of the amount of drug inside the canister.

## Description

### Technical Field

This invention relates to an aerosol can sample content extractor apparatus for determining of drug content (assay) and water content or moisture content of aerosol formulation from pressurized metered dose inhalers (MDI).

### Background Art

Pressurized metered dose inhalers have grown in popularity since their market introduction in the late 1950s and they are expensively used for a variety of diseases such as asthma, chronic obstructive pulmonary disease (COPD) and other lung diseases.

Pressurized metered dose inhalers are compact drug delivery systems where the active ingredient is dispersed or solubilised in liquefied propellant to atomize precisely metered volume of a medicinal formulation into particles, which are small enough to penetrate deep into the patient's lungs.

Pressurized MDIs formulation consists of a drug solution or suspension of propellant which is liquefied in canister, a metering valve and actuator in the mouthpiece.

In pressurized metered dose inhalers (pMDI), high vapour pressure of CFCs or HFAs are used as propellants. Formerly used chlorofluorocarbons are replaced by environmentally friendly hydro fluorocarbons respectively HFA 134a (1, 1, 1, 2-tetrafluoroethane) and H FA 227 (1, 1, 1, 2, 3, 3, 3- heptafluoropropane).

The vapour pressure of a propellant is defined as the pressure of the vapour in thermodynamic equilibrium with its condensed phases in a closed system. For example, HFA 134a has characteristic features of high vapour pressure of 666.1 kPa at 25°C and low boiling point of -26.3°C at atmospheric pressure.

A propellant with vapour pressure exceeding atmospheric pressure is preferable by reason of rapid vaporization by force through the dose metering. Even as the product is used up, there is always a vapour above the liquefied propellant which produces the same pressure independently from the remaining amount of product in the aerosol.

To characterize the product and to ensure the quality and development of metered dose inhalers, it is necessary to determine the chemical test parameters and conduct required stability tests for the formulation. The test parameters are listed as follows; aerosolization, appearance, colour, identification, microbial limit, water/moisture content, dehydrated alcohol content, wet content fill weight, drug content (assay), impurities and degradation product, dose content uniformity, dose content uniformity, dose content uniformity through container life, particle size distribution, microscopic evaluation, spray pattern and plume geometry, leak rate, pressure testing and valve delivery.

The physicochemical properties which are defined by mentioned parameters are important to provide continuity of product quality and reproducible performance of all batches of the drug substance.

Related analytical tests which are required by the pharmacopeia are routinely performed and necessitate repetitive manual sample preparation steps. All inhaler's analytical testing are determined and should be performed conformably to all critical specification listed in USP 601 or EP section 2.9.18.This type of apparatus appears in the European Pharmacopoeia under Dosage Forms-Preparation for Inhalation <0671 > and in the US Pharmacopoeia under section<601>.

Implementation of regular pharmacopoeias tests has led to the development of various devices and apparatus to facilitate execution of required analysis with accurate methods.

Accurate sampling the delivered dose is achievable by dosage unit sampling apparatus. Hence, DUSA (Dosage uniformity sample apparatus) is designed by Charles Thiel to perform tests specified by the relevant compendial standards such as "total number of discharges per container or inhaler" and "dose uniformity".

Furthermore, delivered dose uniformity over the entire content of metered inhaler is performed by Dosage Unit Sampling Apparatus Shaker which is designed to be used in metered dose inhalers dose uniformity testing where 10 doses from a single inhaler is captured for testing dose uniformity throughout the entire metered dose inhaler canister.

Besides, moisture is known to be particularly problematic for HFA propellant based metered dose inhalers. The moisture content of the metered dose inhaler is determined by using an automated Karl Fisher coulometric titrator. Both moisture ingress and moisture content must be analysed and followed throughout the stability period.

WO 01/036967 (GLAXO GROUP LIMITED) 25.05.2001 discloses an automated aerosol can content analyzer workstation. The aerosol can is placed in an extraction chamber where the content of aerosol can is expelled. Then a rinsing fluid is delivered to ruptured aerosol can to extract the content.

WO 03/076907 (SOLVAY FLUOR UND DERIVATIVE GMBH) 18.09.2003 discloses an apparatus for determining the physical and/or chemical parameters of an aerosol formulation from metered dose inhalers. The apparatus of the invention makes it possible to transfer the contents of the cartridge into the measuring chamber where a pH-measuring or a conductivity- measuring or a moisture-measuring electrode or an ion-sensitive electrode is placed.

Although several attempts have been made to design particular equipment that allows a direct on situ measurement of high pressure, mentioned devices do not make possible to expel fully and appropriately to analyse as whole the content of the canister.

### Summary of invention

This invention relates to an aerosol can sample content extractor apparatus for determining of drug content (assay) and water content or moisture content of aerosol formulation from pressurized metered dose inhalers (MDI). The device allows the accurate determination of the amount of drug inside the canister.

### Technical Problem

In continuous manufacturing of pressurized metered dose inhalers, high quality production is crucially important to be conducted under good technical and safety standards. Batch-to-batch consistency of the process should be ensured and release of inadequate dose product is aimed to be avoided.

To obviate possible problems such as the inadequate dose that may occur during production process, related troubleshooting should be taken into consideration by formulation scientist.

Moreover, analysis of dose content assay or water content /moisture content which is based on exact determination of the amount of the drug inside the canister is especially needed to provide evidence to support product quality and shelf life stability before market release of product.

H FA 134a has the feature of high vapour pressure of 666.1 kPa at 25°C.

In under normal conditions HFA134 is found in gas state and it can only liquefy in -70°C of temperature or 6 bar of pressure. Specific conditions of temperature of -70°C or pressure of 6 bars which are generally difficult to achieve in laboratory, should be set to allow experimental measurements in liquefied state HFA containing pressurized metered dose inhalers.

The amount of the drug inside the canister and water content /moisture content should be appropriately analysed and determined. However, analysis of pressurized metered dose inhalers is very limited and difficult with existing techniques due to low boiling point and high vapour pressure of HFA134.

The drug product content testing of aerosol canister is currently performed manually by chilling the aerosol canister and the contents thereof in a dry ice bath. After the contents of the aerosol can have been sufficiently chilled to reduce internal pressure, aerosol can is then opened by a suitable cutting device such as a specialized electric can opener. The aerosol can is then placed into a glass flask and the propellant therein is allowed to evaporate from the opened can. After the propellant has evaporated, the active drug is extracted utilizing a suitable solvent. Liquid handling is then performed on the extraction in order to ready the final sample to be analyzed by UV or HPLC, depending on the drug product. The transfer process of drug content should be performed in one time and step. Hereafter UV or HPLC testing will determine the amount of the drug content in the aerosol can be tested.

The emptying of aerosol container is a labour intensive operation for which a great deal of effort is required. And in addition, there is an element of hazard to the technician.

As for moisture determination, the potential of moisture inside the canister induces the instability and particle aggregation in aqueous suspension. Consequently, both moisture content and moisture ingress rate are carefully analysed and followed throughout the stability period.

Fluorinated hydrocarbons, preferably hydrofluoroalkanes (HFAs) propellants (tetrafluoroethane HFA134a or heptafluooropropane HFA227 or HFA mixture) are especially known to have higher affinity for moisture than the formerly used chlorofluorocarbon propellants. Based on this fact, it is likely that HFA based MDIs have tendency to increased water ingress rate.

On these grounds, moisture ingress and content are of great importance in terms of stability. Moisture ingress into MDIs is problematic, especially for hydroscopic drug particles which have high tendency to absorb water and form aggregates. These types of propellants are regarded to be more prone to moisture. In this context, the limit for moisture content should be established based on results seen in stability studies.

Accordingly, the aim of the invention is to overcome the above mentioned problem, by providing an inhaler sampler device for extracting the whole content of the canister.

### Solution to Problem

In the present invention, difficulty in opening the aerosol canister under specific conditions is surpassed an aerosol can sample content extractor apparatus for analyzing aerosol canister content.

Invented aerosol can sample content extractor (apparatus) enables direct extraction of the aerosol can content to volumetric flask in one step without necessity of canister opening under pressure. This novel apparatus guarantees more reliable results in a shorter amount of time than a traditional methods.

### Brief description of drawings

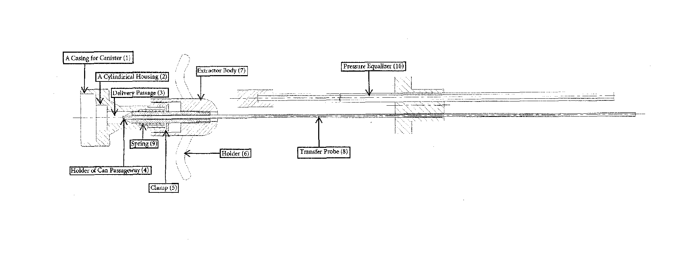

According to Figure 1, aerosol can sample content extractor apparatus comprises a canister casing or a fitting adaptor(1), an aerosol canister or cylindrical housing(2), a delivery passage(3), a holder of can passageway (4), a ferrule(5), a holder(6), extractor body(7) and transfer probes(8), spring (9), pressure equalizer(10).

The present invention will become more fully understood from the detailed description herein and the accompanying drawing which are provided by the way of illustration only and are not to be regarded as limiting the scope of the invention.

### Description of embodiments

In the present invention, inventors provide an aerosol can sample extractor which is configured to collect sample from aerosol canister. The aerosol drug sample is extracted in an automated manner from the canister.

It is an object of the present invention is to provide an aerosol can sample extractor with which it is possible to expel the whole aerosol can content into a volumetric flask total aerosol formulation without loss for dose content analysis and water/moisture content analysis of pressurized metered dose inhalers.

This object of the invention is achieved in determining chemical and physical parameters of pressurized metered dose inhaler.

The aerosol can sample extractor is designed with a simple and efficient structure for emptying the aerosol can content without any loss of drug.

The heading of sample extractor apparatus is designed in a manner to prevent all contact and outside intervention. The extraction is performed in a closed system.

As per Figure 1, aerosol can sample content extractor apparatus comprises a canister casing or a fitting adaptor(1), an aerosol canister or cylindrical housing(2), a delivery passage(3), a holder of can passageway (4), a ferrule(5), a holder(6), extractor body(7) and transfer probes(8), spring (9), pressure equalizer(10).

The aerosol canister is inserted in canister casing (1).Cylindrical housing (2) is the best fit for aerosol valve body.

Delivery passage (3) is fitted to aerosol valve stem. The delivery passage is in fluid communication allowing the pressurized contents of the aerosol can to pass to transfer probe.

Holder of can passageway (4) is to deplete the aerosol canister. When the valve stem is depressed, the aerosol canister content is expelled to delivery passage by with assistance of piercing element.

A clamp (5) allows user to operate quickly, easily by the extraction movement of aerosol.

In order to empty the canister easily, aerosol can sample extractor has two holders (6) at its both sides which serve to hold apparatus appropriately by the technician.

An extractor body (7) is the main body of aerosol can sample content extractor apparatus.

A suitable length of transfer probe (8) is extended into the volumetric flask and allows transfer of drug solution.

A spring (9) is preferably set out between delivery passage and clamp. It serves pushing the casing for canister and returning it previous position.

Preferably, a pressure equalizer or a second shorter transfer probe (10) serves to equalize pressure differences inside and outside the volumetric flask.

A fitting adaptor is additionally designed to open mechanism of metered dose inhaler and can be changed according to the size of gauge of the mouth portion of canister to be used.

Thus, this invention is not limited to canister type and could be applied to other aerosol canister of different size by altering heading of aerosol can sample extractor.

In the present invention, the aerosol can sample content extractor apparatus is made from chemical-resistant material. Stainless steel is preferable without limiting the scope of the invention.

The present invention provides a novel and efficient method of analysing a dose formulation of a metered dose device with an aerosol can sample (content) extractor, in particular a pressurized metered dose inhaler formulation.

According to embodiment of the invention canister casing can be changeable as per canister size.

A method of analysing the content of a pressurised container comprises the following steps:

1) The aerosol can sample content extractor apparatus is placed and fixed on volumetric flask in an upright manner.

2) Transfer probe of aerosol can sample (content) extractor is immersed correctly into the volumetric flask.

3) The sample aerosol canister to analyse is attached at the end of can sample (content) extractor.

4) The entire sample drug suspension in propellant is taken out with the power of propulsion by preventing loss of drug substance.

5) Aerosol canister sample content is directly transferred to solvent part into the head.

The method and apparatus considerably shortens the time required for analysing the dose content of dose metered devices. Additionally drug content analysis is performed in accordance with pharmacopeial standards.

## Claims

1. An aerosol can sample content extractor apparatus comprising (a) a canister casing (1), (b)an aerosol canister housing (2), (c) delivery passage (3), (d) a holder of can passageway (4), (e) a clamp (5), (f) holders (6), (g) extractor body (7) and (h)transfer probe (8).

2. Apparatus, as claimed in claim 1, is preferably accompanied with a second shorter transfer probe (10) as pressure equalizer and a spring (9).

3. A method of analysing the content of aerosol formulation from pressurized metered dose inhalers (MDI) comprising following steps; a) aerosol can sample content extractor apparatus is placed and fixed on volumetric flask in an upright manner, b) Transfer probe of aerosol can sample (content) extractor is immersed correctly into the volumetric flask, c) The sample aerosol canister to analyse is attached at the end of can sample (content) extractor, d) The entire sample drug suspension in propellant is taken out with the power of propulsion by preventing loss of drug substance, e) Aerosol canister sample content is directly transferred to solvent part into the head.
